(19) Europäisches Patentamt
European Patent Office
Office européen des brevets

(11) EP 1 709 052 B1

(12) # FASCICULE DE BREVET EUROPEEN

(45) Date de publication et mention de la délivrance du brevet:
**27.04.2011 Bulletin 2011/17**

(21) Numéro de dépôt: **05717375.9**

(22) Date de dépôt: **07.01.2005**

(51) Int Cl.:
***C07D 487/08*** (2006.01) ***C07D 519/00*** (2006.01)
***A61K 31/416*** (2006.01) ***A61K 31/551*** (2006.01)
***A61P 25/00*** (2006.01) ***A61P 9/00*** (2006.01)

(86) Numéro de dépôt international:
**PCT/FR2005/000027**

(87) Numéro de publication internationale:
**WO 2005/077955 (25.08.2005 Gazette 2005/34)**

(54) **DERIVES DE 1,4 DIAZABICYCLO 3.2.1 OCTANECARBOXAMIDE, LEUR PREPARATION ET LEUR APPLICATION EN THERAPEUTIQUE**

DERIVATE VON 1,4-DIAZABICYCLO3.2.1OCTANCARBONSÄUREAMID, VERFAHREN ZU DEREN HERSTELLUNG UND DEREN VERWENDUNG IN THERAPEUTIKA

DERIVATIVES OF 1,4-DIAZABICYCLO 3.2.1OCTANECARBOXAMIDE, PREPARATION METHOD THEREOF AND USE OF SAME IN THERAPEUTICS

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **16.01.2004 FR 0400390**

(43) Date de publication de la demande:
**11.10.2006 Bulletin 2006/41**

(73) Titulaire: **Sanofi-Aventis**
**75013 Paris (FR)**

(72) Inventeurs:
- **GALLI, Frédéric**
**F-92420 Vaucresson (FR)**
- **LECLERC, Odile**
**F-91300 Massy (FR)**
- **LOCHEAD, Alistair**
**F-94220 Charenton le Pont (FR)**

(74) Mandataire: **Monain, Patrice et al**
**Sanofi aventis**
**Departement Brevets**
**Base Business**
**46 Quai de la Rapée**
**75601 Paris cedex 12 (FR)**

(56) Documents cités:
**WO-A-01/92261** **WO-A-96/11929**
**WO-A-99/42465** **WO-A-03/044018**
**FR-A- 2 845 388**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 709 052 B1

## Description

La présente invention a pour objet des composés, ligands des récepteurs nicotiniques. Le document WO01/92261 décrit des dérivés de 1, 4-diazabicyclo [3.2.2] nonanebenzoxazole, -benzothiazole et-benzimidazole en tant que ligands des récepteurs nicotiniques. Les composés selon l'invention sont utiles dans le traitement ou la prévention des désordres liés à un dysfonctionnement des récepteurs nicotiniques.

Les composés de l'invention répondent à la formule générale (I)

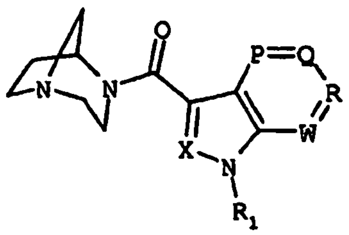

(I)

dans laquelle

X représente un atome d'azote ou un groupe de formule générale C-$R_2$,

P, Q, R et W représentent chacun, indépendamment l'un de l'autre, un atome d'azote ou un groupe de formule générale C-$R_3$,

$R_1$ représente un atome d'hydrogène ou un groupe ($C_1$-$C_6$)alkyle,

$R_2$ représente un groupe ($C_1$-$C_6$)alkyle,

$R_3$ représente un atome d'hydrogène ou d'halogène ou un groupe ($C_1$-$C_6$)alkyle, ($C_1$-$C_6$)alcoxy, nitro, amino, trifluorométhyle, cyano, ou de formule générale -$NR_4R_5$, -$NR_4C(=O)R_5$, -$NR_4C(=O)NR_5R_6$, -$NR_4C(=O)OR_5$, $NR_4S(mO)_2NR_5R_6$, -$OR_5$, -$OC(=O)R_5$, -$OC(=O)OR_5$, -$OC(=O)ONR_4R_5$, -$OC(=O)SR_5$, -$C(=O)OR_5$, $C(=O)R_5$, -$C(=O)NR_4R_5$, $SR_5$, -$S(=O)R_5$, -$S(=O)_2R_5$, -$S(=O)_2NR_4R_6$, ou un groupe phényle éventuellement substitué par un ou plusieurs groupes choisis parmi les atomes d'halogènes et les groupes ($C_1$-$C_6$)alkyle, ($C_1$-$C_6$)alcoxy, nitro, amino, trifluorométhyle, cyano, ou de formule générale -$NR_4R_5$, -$NR_4C(=O)R_5$, -$NR_4C(=O)NR_5R_6$, -$NR_4C(=O)OR_5$, $NR_4S(=O)_2NR_5R_6$, -$OR_5$, -$OC(=O)R_5$, -$OC(=O)OR_5$, -$OC(=O)ONR_4R_5$, -$OC(=O)SR_5$, -$C(=O)OR_5$, -$C(=O)NR_4R_5$, $SR_5$, -$S(=O)R_5$, -$S(=O)_2R_5$, -$S(=O)_2NR_4R_6$, ou $R_3$ représente un groupe choisi parmi les cycles imidazole, pyridine, pyridazine, pyrimidine, pyrazole, pyrazine, triazole, quinoléine, isoquinoléine, tétrazole, furane, thiophène, thiazole, isothiazole, oxazole, isoxazole, pyrrole, tétrahydroquinoléine,

tétrahydroisoquinoléine, indole, benzimidazole, benzofurane, dihydrobenzofurane, cinnoline, indazole, phtalazine, triazine, isoindole, oxadiazole, thiadiazole, furazane, benzofurazane, benzothiophène,

dihydrobenzothiophène, benzotriazole, benzothiazole, benzisothiazole, benzoxazole, benzisoxazole, quinazoline, quinoxaline, naphtyridine, dihydroquinoléine, dihydroisoquinoléine, furopyridine, dihydrofuropyridine, pyrrolopyridine, thiénopyridine, dihydrothiénopyridine, imidazopyridine, pyrazolopyridine, oxazolopyridine, isoxazolopyridine, isoxazolopyridine, thiazolopyridine, isothiazolopyridine, pyrrolopyrimidine, furopyrimidine, dihydrofuropyrimidine, thiénopyrimidine, dihydrothiénopyrimidine, imidazopyrimidine,

pyrazolopyrimidine, oxazolopyrimidine, isoxazolopyrimidine, thiazolopyrimidine, isothiazolopyrimidine, furopyrazine, dihydrofuropyrazine, pyrrolopyrazine, thiénopyrazine, dihydrothiénopyrazine, imidazopyrazine, pyrazolopyrazine, oxazolopyrazine, isoxazolopyrazine, thiazolopyrazine, isothiazolopyrazine, furopyridazine, dihydrofuropyridazine, pyrrolopyridazine, thiénopyridazine, dihydrothiénopyridazine, imidazopyridazine, pyrazolopyridazine, oxazolopyridazine, isoxazolopyridaziné, thiazolopyridazine ou isothiazolopyridazine,

$R_4$, $R_5$ et $R_6$ représentent chacun, indépendamment l'un de l'autre, un atome d'halogène ou un groupe ($C_1$-$C_6$)alkyle linéaire ou ramifié, ($C_2$-$C_6$)alcényle linéaire ou ramifié ou ($C_2$-$C_6$)alcynyle linéaire ou ramifié, ou un groupe ($C_3$-$C_8$) cycloalkyle, ($C_3$-$C_8$) cycloalkyl ($C_1$-$C_3$)alkyle, ($C_4$-$C_8$)cycloalcényle, ou phényle,

les groupes de formules générales $NR_4R_5$ et $NR_5R_6$ pouvant former, avec l'atome d'azote qui les porte, un groupe choisi les groupes aziridinyle, azétidinyle, pyrrolidinyle, pipéridinyle, azépinyle, pipérazinyle, morpholinyle, thiomorpholinyle, pyrrolinyle, indolinyle, pyrazolinyle, pyrazolidinyle, imidazolinyle, 3*H*-indolyle, quinuclidinyle et quinolizinyle.

Les composés de l'invention peuvent exister à l'état de bases ou de sels d'addition à des acides, d'hydrates ou de solvats.

Les cycles de diazabicyclooctane comportant un atome de carbone asymétrique, les composés de l'invention peuvent exister à l'état d'énantiomères purs ou de mélanges d'énantiomères. Les énantiomères peuvent être séparés par des méthodes connues de l'homme du métier, telles que séparation par cristallisation fractionnée de sels diastéréoisomères d'acides chiraux, ou séparation par chromatographie sur support chiral.

Conformément à l'invention, on peut préparer les composés de formule générale (I) par un procédé illustré

par le schéma 1 qui suit.

On fait réagir le 1,4-diazabicyclo[3.2.1]octane de formule (II) avec un composé de formule générale (III) dans laquelle X, P, Q, R, W et $R_1$ sont tels que définis ci-dessus, en présence d'un agent de couplage tel que, par exemple, le *N*,*N*'-carbonyldiimidazole, dans un solvant tel que le diméthylformamide. La fonction acide carboxylique présente sur le composé de formule générale (III) peut aussi, dans une étape préalable, être transformée en fonction chlorure d'acide, pour réagir avec le 1,4-diazabicyclo[3.2.1]octane dans un solvant tel que le dichloroéthane.

Schéma 1

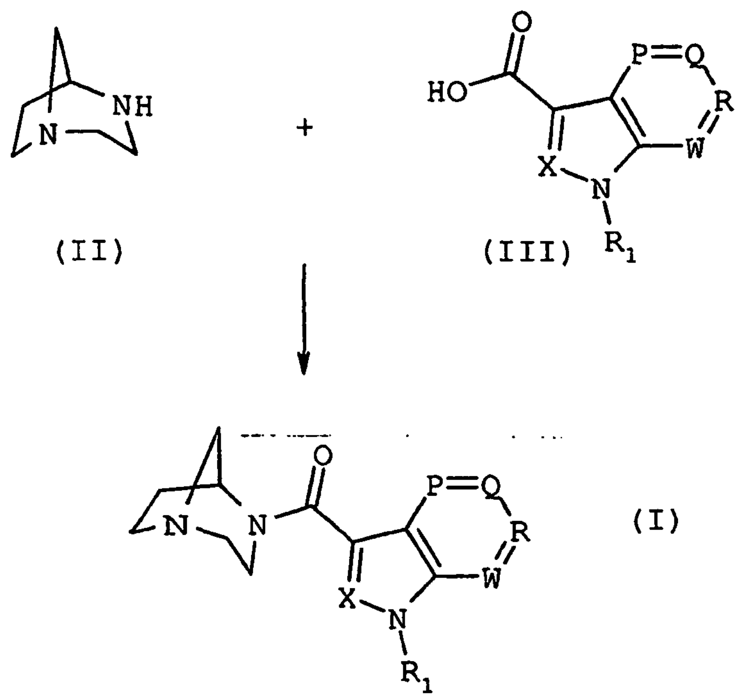

(II) (III) (I)

Alternativement, les composés de formule générale (I) peuvent être préparés par un procédé illustré par le schéma 2 qui suit.

On fait réagir le 1,4-diazabicyclo[3.2.1]octane de formule (II) avec un composé de formule générale (IV), dans laquelle X, P, Q, R, W et $R_1$ sont tels que définis ci-dessus et Z représente un atome de brome ou d'iode, en présence de monoxyde de carbone et d'un catalyseur au palladium tel que, par exemple, le bis(triphénylphosphino)-dichloropalladium, et d'une base telle que, par exemple, la triéthylamine, dans un solvant tel que, par exemple, le diméthylformamide.

Schéma 2

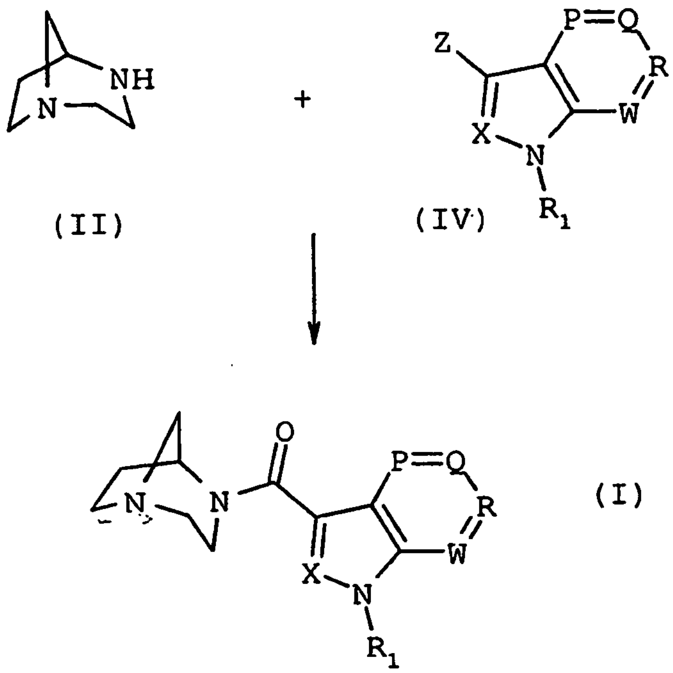

(II) (IV) (I)

Les composés de formule générale (III) sont disponibles dans le commerce ou accessibles par des méthodes décrites dans la littérature, comme par exemple dans Can. J. Chem. 1988, 66, 420-8.

Les composés de formule générale (IV) sont disponibles dans le commerce ou accessibles par des méthodes décrites dans la littérature, comme par exemple dans J. Het. Chem. 1983, 475.

La préparation du 1,4-diazabicyclo[3.2.1]octane est décrite dans J. Med. Chem. 1977, 20, 1333.

Les exemples suivants illustrent en détail la préparation de quelques composés selon l'invention. Les microanalyses élémentaires et les spectres IR et RMN confirment les structures des composés obtenus. Les numéros des composés indiqués entre parenthèses dans les titres correspondent à ceux du tableau donné plus loin. Dans les noms des composés, le tiret "-" fait partie du mot, et le tiret "-" ne sert que pour la coupure en fin de ligne ; il est à supprimer en l'absence de coupure, et ne doit pas être remplacé par un tiret normal ou par un espace.

Exemple 1 (Composé N°2).

Chlorhydrate de 3-(1,4-diazabicyclo[3.2.1]oct-4-ylcarbonyl)-1*H*-indazole 1:1.

Dans un réacteur de 50 ml on introduit 0,165 g (1,02 mmoles) d'acide 1-*H*-indazole-3-carboxylique et 1 ml de chlorure de thionyle, on chauffe le mélange au reflux pendant 1 h 30 et on le concentre sous pression réduite. On ajoute alors 1,2 ml de pyridine et 0,30 g (2,67 mmoles) de 1,4-diazabicyclo[3.2.1]octane et on chauffe le mélange au reflux pendant 1 h 30.

On évapore le solvant sous pression réduite, on reprend le résidu dans 1 ml de chloroforme et on purifie le résidu par chromatographie sur colonne de gel de silice en éluant avec un mélange 70/30/3 d'acétate d'éthyle, méthanol et ammoniaque.

On obtient 0,16 g de produit que l'on dissout dans 10 ml d'acétone avant d'ajouter 0,47 ml d'une solution 5N d'acide chlorhydrique dans l'alcool isopropylique. On collecte les cristaux obtenus (0,17g) par filtration et on les sèche sous pression réduite.

Point de fusion : 286-287°C.

Exemple 2 (composé N°3).

Bromhydrate de 6-chloro-3-(1,4-diazabicyclo[3.2.1]oct-4-ylcarbonyl)1-*H*-indazole 1:1

Dans un réacteur de 10 ml on introduit successivement 0,25 g (0,9 mmole) de 3-iodo-6-chloro-1*H*-indazole, 0,09 g (0,13 mmole) de bis(triphénylphosphino)dichloropalladium, 0,25 g (2,24 mmoles) de 1,4-diazabicyclo[3.2.1]octane et 0,31 ml (2,24 mmoles) de triéthylamine en solution dans 1 ml de diméthylformamide. On purge ensuite le mélange avec du monoxyde de carbone et on chauffe à 70°C pendant 8 h. On verse le milieu réactionnel dans 10 ml d'une solution aqueuse saturée de chlorure d'ammonium et on extrait la phase aqueuse par du chloroforme. On sèche les phases organiques, on les filtre et on les concentre sous pression réduite. On purifie le résidu par chromatographie sur colonne de gel de silice en éluant avec un mélange 90/10/1 de chloroforme, méthanol et ammoniaque.

On obtient ainsi 0,2 g de produit que l'on met en solution dans 1 ml d'alcool isopropylique pour ajouter 0,13 ml d'une solution 5N d'acide chlorhydrique dans l'alcool isopropylique.

On collecte les cristaux obtenus (0,076g) par filtration et on les sèche sous pression réduite.

Point de fusion : 285-286°C.

Exemple 3 (Composé N°1).

Bromhydrate de 3-(1,4-diazabicyclo[3.2.1]oct-4-ylcarbonyl)-6-méthyl-1*H*-pyrazolo[3,4-b]pyridine 2:1.

Par analogie avec l'exemple 2 on fait réagir 0,7 g (3,3 mmoles) de 3-bromo-6-méthyl-1*H*-pyrazolo[3,4-b]pyridine avec 1,1 g (9,9 mmoles) de 1,4-diazabicyclo[3.2.1]octane en présence de 0,35 g (0,5 mmole) de bis(triphénylphosphino) dichloropalladium et 2,3 ml de triéthylamine dans 10 ml de diméthylformamide dans les conditions décrites pour l'exemple 1, et on obtient 0,21 g de produit, que l'on dissout dans 20 ml d'acétone pour ajouter 0,27 ml d'une solution 5,7N d'acide bromhydrique dans l'acide acétique. On collecte les cristaux de dibromhydrate par filtration et on les sèche sous vide.

Point de fusion : 290-291°C.

Exemple 4 (composé N°4).

Bromhydrate de 3-(1,4-diazabicyclo[3.2.1]oct-4-ylcarbonyl)-5-fluoro-1-*H*-indazole 2:1.

Par analogie avec l'exemple 2 on fait réagir 0,23 g (0,88 mmoles) de 3-iodo-5-fluoro-1*H*-indazole avec 0,25 g (2,19 mmoles) de 1,4-diazabicyclo[3.2.1]octane en présence de 0,092 g (0,13 mmole) de bis(triphénylphosphino)dichloro palladium et 0,3 ml de triéthylamine dans 1 ml de diméthylformamide dans les conditions décrites pour l'exemple 2. On obtient 0,136 g de produit que l'on dissout dans 20 ml d'acétone, et on ajoute 0,18 ml d'une solution 5,7N d'acide bromhydrique dans l'acide acétique. On collecte les cristaux de dibromhydrate par filtration et on les sèche sous vide.

Point de fusion : 283-284°C.

Le tableau qui suit illustre les structures chimiques et les propriétés physiques de quelques composés de l'invention.

Dans la colonne "Q", "Me" désigne un groupe méthyle et "Ms" désigne un groupe méthanesulfonyle.

Dans la colonne "St.", "(+/-)" désigne un racémate, "(+)" et "(-)" désignent les énantiomères dextrogyre et lévogyre, respectivement.

Dans la colonne "Sel", "-" désigne un composé à l'état de base, "HBr" désigne un bromhydrate, "HCl" désigne un chlorhydrate et "ox." désigne un oxalate, ou éthanedioate.

Tableau

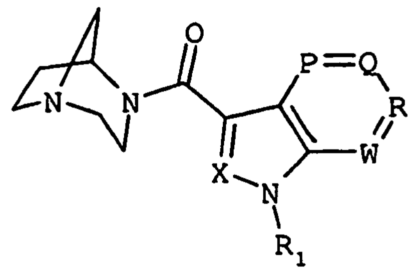

(I)

| N° | X | P | Q | R | W | $R_1$ | St. | Sel | F (°C) |
|---|---|---|---|---|---|---|---|---|---|
| 1 | N | CH | CH | $C-CH_3$ | N | H | (+/-) | HBr | 290-291 |
| 2 | N | CH | CH | CH | CH | H | (+/-) | HCl | 286-287 |
| 3 | N | CH | CH | C-Cl | CH | H | (+/-) | HBr | 285-286 |
| 4 | N | CH | C-F | CH | CH | H | (+/-) | HBr | 283-284 |
| 5 | N | CH | CH | $C-CH_3$ | CH | H | (+/-) | HBr | 299-300 |
| 6 | N | CH | C-OMs | CH | CH | H | (+/-) | ox. | 272-273 |
| 7 | N | CH | C-Cl | CH | CH | H | (+/-) | - | 240-241 |
| 8 | N | CH | C-OMe | CH | CH | H | (+/-) | - | 168-170 |
| 9 | N | CH | CH | CH | N | H | (+/-) | Ox. | 210-211 |
| 10 | N | CH | CH | $C-CH_3$ | N | H | $[\alpha]_D^{20} = -73,9$ (c=0,8 MeOH) | HCl | 327-329 |
| 11 | N | CH | CH | $C-CH_3$ | N | H | $[\alpha]_D^{20} = +70,2$ (c=1 MeOH) | HCl | 328-330 |

Les composés de l'invention ont fait l'objet d'essais pharmacologiques qui ont mis en évidence leur intérêt comme substances actives de médicaments.

Ainsi ils ont été étudiés quant à leur affinité vis-à-vis des récepteurs nicotiniques contenant la sous-unité $\alpha_4\beta_2$ selon les méthodes décrites par Anderson et Arneric dans Eur. J. Pharmacol. 1994, 253, 261 et par Hall et coll. dans Brain Res. 1993, 600, 127.

On décapite des rats mâles Sprague Dawley de 150 à 200 g et on prélève rapidement la totalité du cerveau, on l'homogénéise dans 15 volumes d'une solution de sucrose à 0,32 M à 4°C, puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, on le remet en suspension dans 15 ml d'eau bidistillée et on le centrifuge encore une fois à 40000 G avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de $[^3H]$-cytisine à 1 nM dans un volume final de 500 µl de tampon, en présence ou en absence de composé à tester. On arrête la réaction par filtration sur des filtres Whatman GF/B™ préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de $[^3H]$-cytisine, puis on calcule la $CI_{50}$, concentration de composé qui inhibe 50% de la liaison spécifique.

Les $CI_{50}$ des composés de l'invention les plus affins se situent entre 1 et 10 µM.

Les composés de l'invention ont aussi été étudiés quant à leur affinité vis à vis des récepteurs nicotiniques contenant la sous unité $\alpha_7$, selon les méthodes décrites par Mark et Collins dans J. Pharmacol. Exp. Ther. 1982, 22, 564 et par Marks et coll. dans Mol. Pharmacol. 1986, 30, 427. On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4 °C, puis on le centrifuge à 1000 G pendant 10 min. On élimine le culot et on centrifuge le surnageant à 8000 G pendant 20 min à 4 °C. On récupère le culot et on l'homogénéise à l'aide d'un broyeur Polytron™ dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de $[^3H]$-$\alpha$-bungarotoxine à 1 nM dans un volume final de 250 µl de tampon HEPES 20 mM, polyéthylènimine 0,05%. On arrête la réaction par filtration sur des filtres Whatman GF/C™ préalablement traités pendant 3 h avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de $\alpha$-bungarotoxine à 1 µM finale ; la liaison non spécifique représente environ 60 % de la liaison totale récupérée sur le filtre. Pour chaque concentration de composé étudié on détermine le pourcentage d'inhibition de la liaison spécifique de $[^3H]$-$\alpha$-bungarotoxine, puis on calcule la $CI_{50}$, concentration de composé qui inhibe 50% de la liaison spécifique.

Les $CI_{50}$ des composés de l'invention les plus affins se situent entre 0,010 et 0,10 µM.

Les $CI_{50}$ de quelques composés spécifiques sont indiqués dans le tableau suivant.

| Composé N° | $CI_{50}$ $\alpha_7$ µM | $CI_{50}$ $\alpha_4\beta_2$ |
|---|---|---|
| 1 | 0,056 | >10 |
| 4 | 0,055 | - |
| 7 | 0,224 | - |

Les résultats qui précèdent montrent que les composés de l'invention sont des ligands sélectifs pour les sous unités $\alpha_7$ du récepteur nicotinique.

Les résultats des divers essais suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus spécifiquement mnésiques, mais également attentionnelles, liées à la maladie d'Alzheimer, au vieillissement pathologique (Age Associated Memory Impairment, AAMI),

au syndrome Parkinsonien, à la trisomie 21 (Down's syndrome), au syndrome alcoolique de Korsakoff, aux démences vasculaires (multi-infarct dementia, MDI).

Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.

Les composés de l'invention peuvent également constituer un traitement curatif ou symptomatique des accidents vasculaires cérébraux et des épisodes hypoxiques cérébraux. Ils peuvent être utilisés dans les cas de pathologies psychiatriques : schizophrénie, dépression, anxiété, attaques de panique, comportements compulsifs et obsessionnels.

Ils peuvent prévenir les symptômes dus au sevrage au tabac, à l'alcool, aux différentes substances induisant une dépendance, telles que cocaïne, LSD, cannabis, benzodiazépines.

Par ailleurs les composés de l'invention peuvent aussi être utilisés pour le traitement de l'ischémie des membres inférieurs, de l'artérite oblitérante des membres inférieurs (PAD : peripheral arterial disease), de l'ischémie cardiaque (angor stable), de l'infarctus du myocarde, de l'insuffisance cardiaque, du déficit de cicatrisation cutanée des patients diabétiques, des ulcères variqueux de l'insuffisance veineuse.

Pour chacune des pathologies précitées, le traitement peut se faire avec l'agent nicotinique seul et/ou en association avec les médicaments de référence indiqués dans la pathologie.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le Lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.

Les techniques de réalisation peuvent être la compression directe, la granulation sèche, la granulation humide ou la fusion à chaud.

Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.

Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylène-glycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se

présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé, à l'état d'énantiomère pur ou de mélange d'énantiomères, répondant à la formule générale (I)

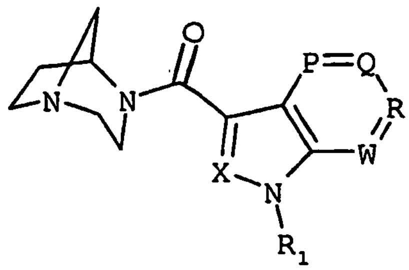

(I)

dans laquelle
X représente un atome d'azote ou un groupe de formule générale C-$R_2$,
P, Q, R et W représentent chacun, indépendamment l'un de l'autre, un atome d'azote ou un groupe de formule générale C-$R_3$,
$R_1$ représente un atome d'hydrogène ou un groupe ($C_1$-$C_6$) alkyle,
$R_2$ représente un groupe ($C_1$-$C_6$) alkyle,
$R_3$ représente un atome d'hydrogène ou d'halogène ou un groupe ($C_1$-$C_6$) alkyle, ($C_1$-$C_6$)alcoxy, nitro, amino, trifluorométhyle, cyano, ou de formule générale $-NR_4R_5$, $-NR_4C\ (=O)R_5$, $-NR_4C\ (=O)NR_5R_6$, $-NR_4C(=O)OR_5$, $NR_4S(=O)_2NR_5R_6$, $-OR_5$, $-OC(=O)R_5$, $-OC(=O)OR_5$, $-OC(=O)ONR_4R_5$, $-OC(=O)SR_5$, $-C(=O)OR_5$, $C(=O)R_5$, $-C(=O)NR_4R_5$, $SR_5$, $-S(=O)R_5$, $-S(=O)_2R_5$, $-S(=O)_2NR_4R_6$, ou un groupe phényle éventuellement substitué par un ou plusieurs groupes choisis parmi les atomes d'halogènes et les groupes ($C_1$-$C_6$)alkyle, ($C_1$-$C_6$)alcoxy, nitro, amino, trifluorométhyle, cyano, ou de formule générale $-NR_4R_5$, $-NR_4C(=O)R_5$, $-NR_9C(=O)NR_5R_6$, $-NR_4C(=O)OR_5$, $NR_4S(=O)_2NR_5R_6$, $-OR_5$, $-OC(=O)R_5$, $-OC(=O)OR_5$, $-OC(=O)ONR_4R_5$, $-OC(=O)SR_5$, $-C\ (=O)\ OR_5$, $-C(=O)NR_4R_5$, $SR_5$, $-S\ (=O)R_5$, $-S\ (=O)_2R_5$, $-S\ (=O)_2NR_4R_6$,
ou bien $R_3$ représente un groupe choisi parmi les cycles imidazole, pyridine, pyridazine, pyrimidine, pyrazole, pyrazine, triazole, quinoléine, isoquinoléine, tétrazole,
furane, thiophène, thiazole, isothiazole, oxazole, isoxazole, pyrrole, tétrahydroquinoléine, tétrahydroisoquinoléine, indole, benzimidazole, benzofurane, dihydrobenzofurane, cinnoline, indazole, phtalazine, triazine, isoindole, oxadiazole, thiadiazole, furazane, benzofurazane, benzothiophène, dihydrobenzothiophène, benzotriazole, benzothiazole, benzisothiazole, benzoxazole, benzisoxazole, quinazoline, quinoxaline, naphtyridine, dihydroquinoléine, dihydroisoquinoléine, furopyridine, dihydrofuropyridine, pyrrolopyridine, thiénopyridine, dihydrothiénopyridine, imidazopyridine, pyrazolopyridine, oxazolopyridine, isoxazolopyridine, isoxazolopyridine, thiazolopyridine, isothiazolopyridine, pyrrolopyrimidine, furopyrimidine, dihydrofuropyrimidine, thiénopyrimidine, dihydrothiénopyrimidine, imidazopyrimidine, pyrazolopyrimidine, oxazolopyrimidine, isoxazolopyrimidine, thiazoloyrimidine, isothiazoloyrimidine, furopyrazine, dihydrofuropyrazine, pyrrolopyrazine, thiénopyrazine, dihydrothiénopyrazine, imidazopyrazine, pyrazolopyrazine, oxazolopyrazine, isoxazolopyrazine, thiazolopyrazine, isothiazolopyrazine, furopyridazine, dihydrofuropyridazine, pyrrolopyridazine, thiénopyridazine, dihydrothiénopyridazine, imidazopyridazine, pyrazolopyridazine, oxazolopyridazine, isoxazolopyridazine, thiazolopyridazine ou isothiazolopyridazine,
$R_4$, $R_5$ et $R_6$ représentent chacun, indépendamment l'un de l'autre, un atome d'halogène ou un groupe ($C_1$-$C_6$) alkyle linéaire ou ramifié, ($C_2$-$C_6$)alcényle linéaire ou ramifié ou ($C_2$-$C_6$)alcynyle linéaire ou ramifié, ou un groupe ($C_3$-$C_8$)cycloalkyle, ($C_3$-$C_8$)cycloalkyl($C_1$-$C_3$)alkyle, ($C_4$-$C_8$)cycloalcényle, ou phényle,
les groupes de formules générales $NR_4R_5$ et $NR_5R_6$ pouvant former, avec l'atome d'azote qui les porte, un groupe choisi les groupes aziridinyle, azétidinyle, pyrrolidinyle, pipéridinyle, azépinyle, pipérazinyle, morpholinyle, thiomorpholinyle, pyrrolinyle, indolinyle, pyrazolinyle, pyrazolidinyle, imidazolinyle, 3H-indolyle, quinuclidinyle et quinolizinyle,

à l'état de base, de solvat ou de sel d'addition à un acide.

2. Médicament, **caractérisé en ce qu'**il consiste en un composé selon la revendication 1.

3. Composition pharmaceutique, **caractérisée en ce qu'**elle contient un composé selon la revendication 1, associé à un excipient.

4. Utilisation d'un composé selon la revendication 1 pour la préparation d'un médicament destiné au traitement des troubles dus aux altérations cognitives et attentionnelles ou des troubles moteurs, des troubles neurologiques, psychiatriques ou destiné à la prévention des symptômes dus au sevrage aux substances induisant une dépendance ou destiné au traitement des pathologies cardiaques, vasculaires, artérielles et veineuses.

## Claims

1. Compound, in the form of a pure enantiomer or enantiomer mixture, conforming to the general formula (I)

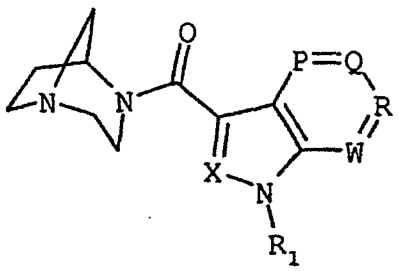

(I)

in which
X represents a nitrogen atom or a group of general formula C-$R_2$,
P, Q, R and W represent each independently of one another a nitrogen atom or a group of general formula C-$R_3$,
$R_1$ represents a hydrogen atom or a ($C_1$-$C_6$)alkyl group,
$R_2$ represents a ($C_1$-$C_6$)alkyl group,
$R_3$ represents a hydrogen or halogen atom or a ($C_1$-$C_6$)alkyl, ($C_1$-$C_6$) alkoxy, nitro, amino, trifluoromethyl or cyano group or a group of general formula $-NR_4R_5$, $-NR_4C(=O)R_5$, $-NR_4C(=O)NR_5R_6$, $-NR_4C(=O)OR_5$, $NR_4S(=O)_2NR_5R_6$, $-OR_5$, -OC $(=O)R_5$, $-OC(=O)OR_5$, $-OC(=O)ONR_4R_5$, $-OC(=O)SR_5$, $-C(=O)OR_5$, $C(=O)R_5$, $-C(=O)NR_4R_5$, $SR_5$, -S $(=O)R_5$, $-S(=O)_2R_5$ or- $S(=O)_2NR_4R_6$, or a phenyl group optionally substituted by one or more groups selected from halogen atoms and ($C_1$-$C_6$)alkyl, ($C_1$-$C_6$)alkoxy, nitro, amino, trifluoromethyl or cyano groups or groups of general formula $-NR_4R_5$, $-NR_4C(=O)R_5$, $-NR_4C(=O)NR_5R_6$, $-NR_4C(=O)OR_5$, $NR_4S(=O)_2NR_5R_6$, $-OR_5$, $-OC(=O)R_5$, -OC $(=O)OR_5$, $-OC(=O)ONR_4R_5$, $-OC(=O)SR_5$, $-C(=O)OR_5$, $-C(=O)NR_4R_5$, $SR_5$, $-S(=O)R_5$, $-S(=O)_2R_5$ or $-S(=O)_2NR_4R_6$, or $R_3$ represents a group selected from imidazole, pyridine, pyridazine, pyrimidine, pyrazole, pyrazine, triazole, quinoline, isoquinoline, tetrazole, furan, thiophene, thiazole, isothiazole, oxazole, isoxazole, pyrrole, tetrahydroquinoline, tetrahydroisoquinoline, indole, benzimidazole, benzofuran, dihydrobenzofuran, cinnoline, indazole, phthalazine, triazine, isoindole, oxadiazole, thiadiazole, furazan, benzofurazan, benzothiophene, dihydrobenzothiophene, benzotriazole, benzothiazole, benzisothiazole, benzoxazole, benzisoxazole, quinazoline, quinoxaline, naphthyridine, dihydroquinoline, dihydroisoquinoline, furopyridine, dihydrofuropyridine, pyrrolopyridine, thienopyridine, dihydrothienopyridine, imidazopyridine, pyrazolopyridine, oxazolopyridine, isoxazolopyridine, isoxazolopyridine, thiazolopyridine, isothiazolopyridine, pyrrolopyrimidine, furopyrimidine, dihydrofuropyrimidine, thienopyrimidine, dihydrothienopyrimidine, imidazopyrimidine, pyrazolopyrimidine, oxazolopyrimidine, isoxazolopyrimidine, thiazolopyrimidine, isothiazolopyrimidine, furopyrazine, dihydrofuropyrazine, pyrrolopyrazine, thienopyrazine, dihydrothienopyrazine, imidazopyrazine, pyrazolopyrazine, oxazolopyrazine, isoxazolopyrazine, thiazolopyrazine, isothiazolopyrazine, furopyridazine, dihydrofuropyridazine, pyrrolopyridazine, thienopyridazine, dihydrothienopyridazine, imidazopyridazine, pyrazolopyridazine, oxazolopyridazine, isoxazolopyridazine, thiazolopyridazine or isothiazolopyridazine ring systems, $R_4$, $R_5$ and $R_6$ represent each independently of one another a halogen atom or a linear or branched ($C_1$-$C_6$)alkyl, linear or branched ($C_2$-$C_6$)alkenyl or linear or branched ($C_2$-$C_6$)alkynyl group, or a ($C_3$-$C_8$) cycloalkyl, ($C_3$-$C_8$) cycloalkyl ($C_1$-$C_3$) alkyl, ($C_4$-$C_8$)cycloalkenyl or phenyl group,
it being possible for the groups of general formulae $NR_4R_5$ and $NR_5R_6$ to form, with the nitrogen atom which carries them, a group selected from aziridinyl, azetidinyl, pyrrolidinyl, piperidinyl, azepinyl, piperazinyl, morpholinyl, thiomorpho linyl, pyrrolinyl, indolinyl, pyrazolinyl, pyrazolidinyl, imidazolinyl, 3H-indolyl, quinuclidinyl and quinolizinyl groups,
in the form of a base, a solvate or an addition salt with an acid.

**2.** Medicament **characterized in that** it consists of a compound according to Claim 1.

**3.** Pharmaceutical composition **characterized in that** it contains a compound according to Claim 1 in combination with an excipient.

**4.** Use of a compound according to Claim 1 for preparing a medicament intended for the treatment of disorders caused by cognitive and attention impairment or motor disorders, neurological or psychiatric disorders, or intended for preventing the symptoms caused by withdrawal from substances which induce dependency, or intended for the treatment of cardiac, vascular, arterial, and venous pathologies.

**Patentansprüche**

**1.** Verbindung in Form eines Enantiomers oder eines Enantiomerengemischs mit der allgemeinen Formel (I) :

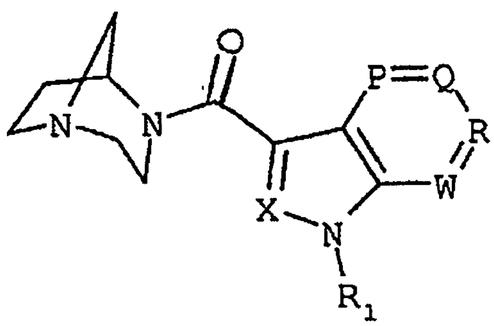

(I)

worin
X für ein Stickstoffatom oder eine Gruppe der allgemeinen Formel C-$R_2$ steht,
P, Q, R und W jeweils unabhängig voneinander für ein Stickstoffatom oder eine Gruppe der allgemeinen Formel C-$R_3$ stehen,
$R_1$ für ein Wasserstoffatom oder eine ($C_1$-$C_6$)-Alkylgruppe steht,
$R_2$ für eine ($C_1$-$C_6$)-Alkylgruppe steht,
$R_3$ für ein Wasserstoff- oder Halogenatom oder eine ($C_1$-$C_6$)-Alkyl-, ($C_1$-$C_6$)-Alkoxy-, Nitro-, Amino-, Trifluormethyl- oder Cyanogruppe oder eine Gruppe der allgemeinen Formel -$NR_4R_5$, -$NR_4C(=O)R_5$, -$NR_4C(=O)NR_5R_6$, -$NR_4C$ $(=O)OR_5$, $NR_4S$ $(=O)_2NR_5R_6$, -$OR_5$, -$OC(=O)R_5$, -$OC(=O)OR_5$, -OC $(=O)ONR_4R_5$, -$OC(=O)SR_5$,- C $(=O)OR_5$, C $(=O)R_5$, -C $(=O)NR_4R_5$, $SR_5$, -S (=0) $R_5$, -$S(=O)_2R_5$ oder -$S(=O)_2NR_4R_6$ oder eine Phenylgruppe, die gegebenenfalls durch eine oder mehrere unter Halogenatomen und ($C_1$-$C_6$)-Alkyl-, ($C_1$-$C_6$)-Alkoxy-, Nitro-, Amino-, Trifluormethyl- oder Cyanogruppen oder Gruppen der allgemeinen Formel -$NR_4R_5$, -$NR_4C(=O)R_5$, -$NR_4C(=O)NR_5R_6$, -$NR_4C$ (=O) $OR_5$, $NR_4S(=O)_2NR_5R_6$, -$OR_5$, -$OC(=O)R_5$, -$OC(=O)OR_5$, -$OC(=O)ONR_4R_5$, -$OC(=O)SR_5$, -$C(=O)OR_5$, -C(=O) $NR_4R_5$, $SR_5$, -$S(=O)R_5$, -$S(=O)_2R_5$ oder -$S(=O)_2NR_4R_6$ ausgewählte Gruppen substituiert ist, steht,
oder $R_3$ für eine unter Imidazol-, Pyridin-, Pyridazin-, Pyrimidin-, Pyrazol-, Pyrazin-, Triazol-, Chinolin-, Isochinolin-, Tetrazol-, Furan-, Thiophen-, Thiazol-, Isothiazol-, Oxazol-, Isoxazol-, Pyrrol-, Tetrahydrochinolin-, Tetrahydroisochinolin-, Indol-, Benzimidazol-, Benzofuran-, Dihydrobenzofuran-, Cinnolin-, Indazol-, Phthalazin-, Triazin-, Isoindol-, Oxadiazol-, Thiadiazol-, Furazan-, Benzofurazan-, Benzothiophen-, Dihydrobenzothiophen-, Benzotriazol-, Benzothiazol-, Benzisothiazol-, Benzoxazol-, Benzisoxazol-, Chinazolin-, Chinoxalin-, Naphthyridin-, Dihydrochinolin-, Dihydroisochinolin-, Furopyridin-, Dihydrofuropyridin-, Pyrrolopyridin-, Thienopyridin-, Dihydrothienopyridin-, Imidazopyridin-, Pyrazolopyridin-, Oxazolopyridin-, Isoxazolopyridin-, Isoxazolopyridin-, Thiazolopyridin-, Isothiazolopyridin-, Pyrrolopyrimidin-, Furopyrimidin-, Dihydrofuropyrimidin-, Thienopyrimidin-, Dihydrothienopyrimidin-, Imidazopyrimidin-, Pyrazolopyrimidin-, Oxazolopyrimidin-, Isoxazolopyrimidin-, Thiazolopyrimidin-, Isothiazolopyrimidin-, Furopyrazin-, Dihydrofuropyrazin-, Pyrrolopyrazin-, Thienopyrazin-, Dihydrothienopyrazin-, Imidazopyrazin-, Pyrazolopyrazin-, Oxazolopyrazin-, Isoxazolopyrazin-, Thiazolopyrazin-, Isothiazolopyrazin-, Furopyridazin-, Dihydrofuropyridazin-, Pyrrolopyridazin-, Thienopyridazin-, Dihydrothienopyridazin-, Imidazopyridazin-, Pyrazolopyridazin-, Oxazolopyridazin-, Isoxazolopyridazin-, Thiazolopyridazin- oder Isothiazolopyridazinringen ausgewählte Gruppe steht,
$R_4$, $R_5$ und $R_6$ jeweils unabhängig voneinander für ein Halogenatom oder eine lineare oder verzweigte ($C_1$-$C_6$)-Alkylgruppe, eine lineare oder verzweigte ($C_2$-$C_6$)-Alkenylgruppe, eine lineare oder verzweigte ($C_2$-$C_6$)-Alkinylgruppe oder eine ($C_3$-$C_8$)-Cycloalkyl-, ($C_3$-$C_8$)-Cycloalkyl-($C_1$-$C_3$)-alkyl-, ($C_9$-$C_8$)-Cycloalkenyl- oder Phenylgruppe stehen, wobei die Gruppen der allgemeinen Formeln $NR_4R_5$ und $NR_5R_6$ mit dem Stickstoffatom, an das sie gebunden sind, eine unter Aziridinyl-, Azetidinyl-, Pyrrolidinyl-, Piperidinyl-, Azepinyl-, Piperazinyl-, Morpholinyl-, Thiomorpholinyl-, Pyrrolinyl-, Indolinyl-, Pyrazolinyl-, Pyrazolidinyl-, Imidazolinyl-, 3H-Indolyl-, Chinuclidinyl- und Chinolizinylgruppen ausgewählte Gruppe bilden können,
in Basen-, Solvat- oder Säureadditionssalzform.

**2.** Arzneimittel, **dadurch gekennzeichnet, daß** es aus einer Verbindung nach Anspruch 1 besteht.

**3.** Pharmazeutische Zusammensetzung, **dadurch gekennzeichnet, daß** sie eine Verbindung nach Anspruch 1 in Kombination mit einem Hilfsstoff enthält.

**4.** Verwendung einer Verbindung nach Anspruch 1 zur Herstellung eines Arzneimittels zur Behandlung von durch kognitive Beeinträchtigungen oder Aufmerksamkeitsbeeinträchtigung verursachten Störungen oder motorischen Störungen, neurologischen Störungen oder psychiatrischen Störungen oder zur Prävention von durch Entzug von abhängigmachenden Substanzen verursachten Symptomen oder zur Behandlung von Herz-, Gefäß-, Arterien- und Venenpathologien.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0192261 A


### Littérature non-brevet citée dans la description


- *Can. J. Chem.,* 1988, vol. 66, 420-8
- *J. Het. Chem.,* 1983, 475
- *J. Med. Chem.,* 1977, vol. 20, 1333
- *Eur. J. Pharmacol.,* 1994, vol. 253, 261
- **Hall.** *Brain Res.,* 1993, vol. 600, 127
- **Mark ; Collins.** *J. Pharmacol. Exp. Ther.,* 1982, vol. 22, 564
- **Marks.** *Mol. Pharmacol.,* 1986, vol. 30, 427